# EUROPEAN PATENT APPLICATION

(11) **EP 1 353 001 A1**
(43) Date of publication of application: **15.10.2003**
(21) Application number: 02425223.1
(22) Date of filing: 11.04.2002
(51) Int. Cl.: D04H 1/70, D04H 13/00, A61F 13/15, A61F 13/515

(54) **Absorbent article**

(71) Applicant: Main S.p.A., 37050 S. Maria di Zevio VR (IT)
(72) Inventor: Milesi, Domenico, 00136 Roma (IT); Bubbico, Cesare, 75024 Montescaglioso MT (IT); Bertocchi, Gabriele, 65125 Pescara (IT)
(74) Representative: Leone, Mario

(57) **Abstract**

Absorbent article, comprising a permeable topsheet (102), an impermeable backsheet (105) and an absorbent body (104) located between said topsheet (102) and said backsheet (105), made of a single structured member comprising two or more intermediate layers (111, 112, 113) interconnected by thermal melting.

## Description

The present invention refers to an absorbent article, such as a diaper, a training pant, a sanitary napkin, a wound dressing, a breast pad, or the like.

Absorbent articles of the abovementioned kind are usually employed to absorb body fluids such as urine, blood or breast milk. They usually comprise a liquid pervious topsheet, facing the wearer during use and in the form of a nonwoven material or a perforated film or of a combination thereof.

Moreover, absorbent articles are known that incorporate a liquid acquisition layer between the topsheet and the absorbent body. Said acquisition layer is capable of quickly receiving large amounts of fluid, of distributing and temporarily storing the latter prior to the absorption thereof by the underlying absorbent body.

The function performed by the acquisition layer is especially important in view of the manufacturing of ever thinner and more compact articles, yet having a high absorbing capacity and concomitantly a high absorbing rate.

However, to date the materials used as acquisition layers in absorbent products, though functional, are particularly expensive.

Other problems are entailed in the 'on-line' process for the application of said acquisition layers above the absorbent bodies.

Said application is usually carried out via the use of adhesive substances, that, unavoidably, lead to the formation of barriers to fluids through the acquisition layer. On the other hand, in the absence of adhesive substances, irregularities in the surface of said applied acquisition layer and of the absorbent body could generate air spaces, thereby limiting the passage of fluids.

Another problem of the known art lies in that the traditional materials used for the manufacturing of the permeable topsheet, usually a nonwoven material of synthetic fibres, often exhibit a fluid acquisition rate lower than that of the acquisition layer. Therefore, the fluid to be absorbed can leak from the article prior to reaching the acquisition layer.

Sometimes this problem is solved using a perforated material to manufacture the topsheet, a material having a high liquid permeability.

However, such a material often causes further problems, as fibre particles from the acquisition layer can penetrate the holes of the perforated material, irritating the user's skin.

The absorbent body of the known absorbent articles, in the thin and compact structures of the state of the art, are typically manufactured by combining layers of airlaid absorbent nonwoven and of superabsorbent materials.

The layers of superabsorbent materials are usually fixed to the other layers by means of hot melt adhesives that entail the drawback of reducing the overall performance of the absorbent body. This is so since, however located, said adhesives tend to cover more or less extended portions of the absorbents, inactivating them.

Object of the present invention is to solve the abovementioned problems of the known art, providing an absorbent article comprising a permeable topsheet, an impermeable backsheet and an absorbent body located between said topsheet and said backsheet, characterised in that said absorbent body is made of a single structured member comprising two or more intermediate layers interconnected by thermal melting, said structured member being thermally fixed to said topsheet and backsheet.

A further object of the present invention is to provide a process for the manufacturing of an absorbent article, characterised in that it comprises the steps of:
- providing a permeable topsheet;
- providing an impermeable backsheet;
- forming a single absorbent body comprising two or more thermally interconnected intermediate layers;
- placing said absorbent body between said topsheet and said backsheet; and
- thermally fixing said absorbent body to said topsheet and to said backsheet.

A first advantage of the absorbent article according to the present invention lies in that it fully manages the fluids with no need to overlap an acquisition layer to the absorbent body.

A second advantage of the present invention lies in that it provides a low distribution of the fluids in direct contact with the permeable topsheet. This is particularly advantageous in the case of topsheets made of perforated or breathable materials, as it improves the overall performances thereof.

A further advantage of the present invention lies in the substantial simplification of the traditional manufacturing process, which is made less complex and more cost-effective.

Further advantages, features and modes of employ of the present invention will be made apparent in the following detailed description of an embodiment thereof, given by way of a non-limiting example, making reference to the figures of the attached drawings, wherein:
figure 1 is a cross-sectional view of a known absorbent article;
figure 2 is a cross-sectional view of an absorbent article according to the present invention; and
figure 3 is a block diagram of a plant for the manufacturing of absorbent articles according to the present invention.

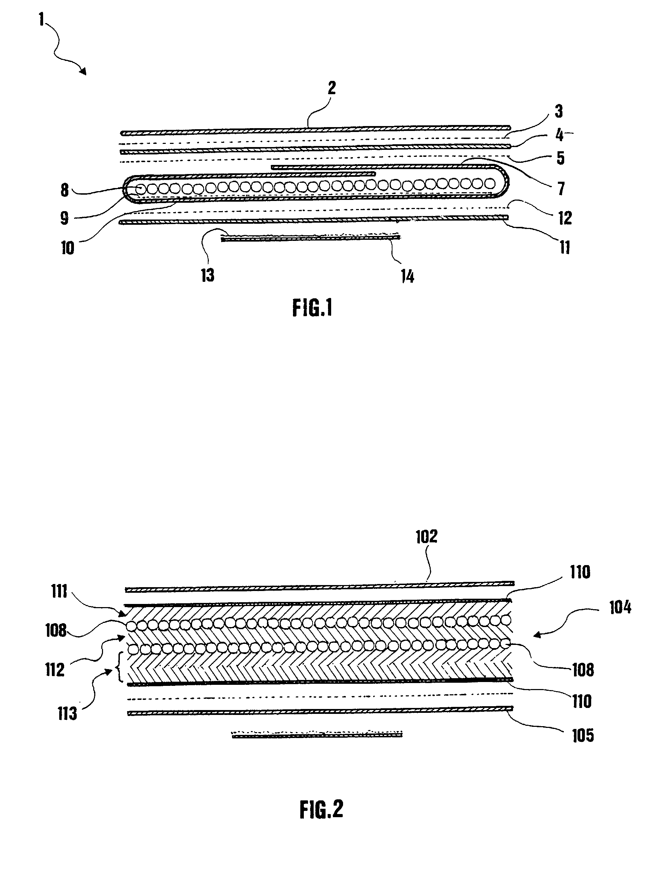

With reference to figure 1 a known absorbent article is illustrated.

An absorbent article 1 consists of a topsheet 2, overlapped to an acquisition layer 4.

The topsheet 2 is fixed to the liquid acquisition layer 4 by means of a layer 3 of adhesive material, usually of hot melt type.

The acquisition layer 4 is in turn fixed to an absorbent body 6 through a further adhesive layer 5.

The absorbent body 6 is usually obtained by folding an absorbent airlaid layer 7 around a polymer superabsorbent layer 8.

The superabsorbent layer 8 is in turn fixed by means of adhesives 9 on a section 10 of the absorbent airlaid layer 7.

Below the absorbent body 6, at the back surface thereof, a barrier layer 11 is applied by adhesive means 12.

Moreover, in some cases, e.g. for sanitary absorbent articles, to the outer surface of said barrier 11 an adhesive layer 13 is applied in order to enable the fixing of the article to underwear items.

In this case, the adhesive 13 is protected by a silicone film or paper strip 14.

Next, figure 2 is a sectional view of a preferred embodiment of an absorbent article according to the present invention.

A topsheet 102 is preferably made of non-perforated nonwoven.

Alternatively, the sheet 102 could be made of perforated nonwoven or of perforated film, or even by lamination of a perforated film with one or more nonwoven layers.

The topsheet 102 is fixed at its bottom surface to a structured member 104, with a thermobonding process, without adhesives.

The thermobonding process is enabled by the structural homogeneity between the synthetic fibres forming the topsheet 102 and the top surface of the structured member 104.

The structured member 104 according to the present invention is manufactured by means of a mixed process.

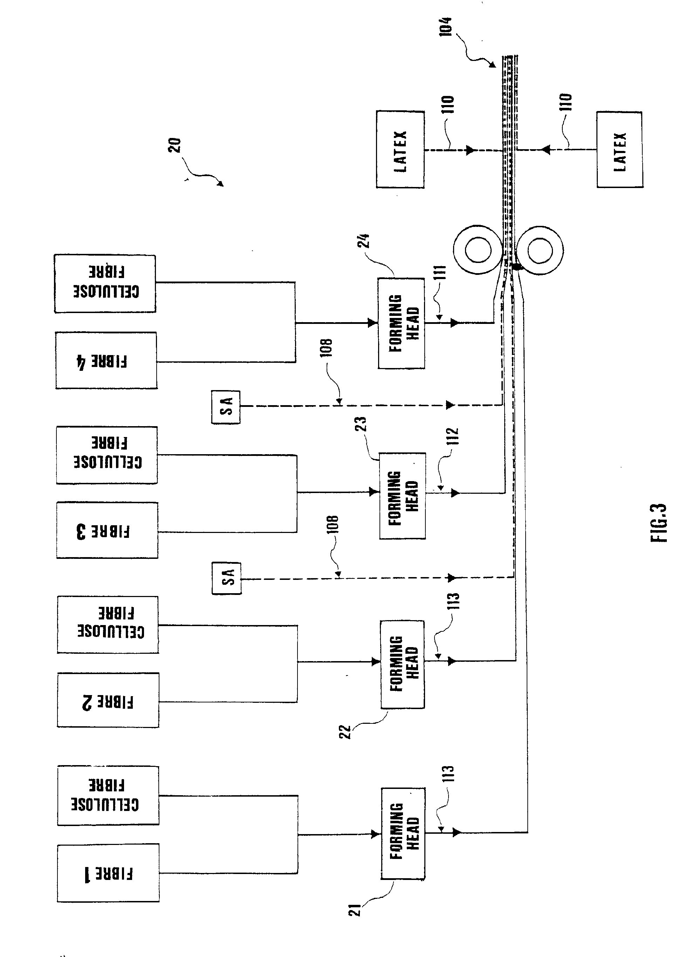

Figure 3 sketches a typical plant for the manufacturing of the structured member 104, according to the process of the present invention.

According to the preferred embodiment, a plant 20 comprises four forming heads 21, 22, 23, 24.

However, also a lower number of forming heads, e.g. three, may be used, according to the specific manufacturing needs.

Each of said heads is fed with an advantageously selected mixture of synthetic fibres and cellulose fibres.

Preferred synthetic fibres can be bicomponent fibres of the PP (core) /PE (sheath) type or of the PES(core)/CoPE(sheath) type, of different denier and fibre length, according to the specific application.

Preferred cellulose fibres are obtained from the defibering of untreated cellulose pulp.

The forming head 24 is apt to form a first intermediate layer 111 of the three intermediate layers forming the structured member 104.

According to a preferred embodiment of the present invention, the forming head 24 is preferably fed with a mixture of fibres comprising from the 50% to the 100% of its total weight (b/w) of thermoplastic fibres, preferably of the bicomponent or the like type, the rest consisting of cellulose fibres.

According to a preferred embodiment, the percent of thermoplastic fibres is of at least the 90% b/w.

The fibres of the first intermediate layer 111 have a denier of at least 3 dtex and a length of at least 3 mm. According to the preferred embodiment, the thermoplastic fibres of the first intermediate layer 111 have a denier of 10 dtex and a length of at least 6 mm.

The forming head 23 is apt to form a second intermediate layer 112 of the three intermediate layers forming the structured member 104.

The intermediate forming head 23 is preferably fed with a mixture of fibres such that at least the 70% b/w of the mixture consist of thermoplastic fibres, preferably bicomponent or the like, and the rest consist of cellulose fibres.

According to the preferred embodiment, the thermoplastic fibres of the second intermediate layer 112 have a denier of at least 3 dtex and a length of at least 3 mm.

Alternatively, the forming head 23 may be fed with a mixture of fibres such that at least the 90% b/w of the mixture consist of thermoplastic fibres, preferably bicomponent or the like, and the rest consist of cellulose fibres. In this case, the thermoplastic fibres of the second intermediate layer 112 have a denier of at least 3 dtex and a length of at least 6 mm.

The forming heads 21, 22 are apt to form a third intermediate layer 113 of the three intermediate layers forming the structured member 104.

Each one of the forming heads 21, 22 is fed with a mixture of fibres such that at least the 15% b/w thereof consist of thermoplastic fibres, preferably bicomponent or the like, and the rest consist of cellulose fibres.

According to the preferred embodiment, the thermoplastic fibres of the third intermediate layer 113 have a denier of at least 3 dtex and a length of at least 3 mm.

Advantageously, a predetermined quantity of superabsorbent particles 108 is introduced in the structured member 104, through a step of deposing the desirable quantity above one or more of the layers released by the individual forming heads.

In the example of figure 3, superabsorbent particles are introduced above the layer released by the forming head 22 and above the layer released by the forming head 23.

Alternatively, the superabsorbent particles can be directly admixed to the other fibres in each forming head.

Alternatively, there may be used superabsorbent fibres directly fed to one or more of the forming heads.

The required strength of the structured member 104 is attained by means of the interconnection created by the melting of thermobonding fibres at their points of contact.

Onto one or both of the outer surfaces of the structured member 104 thus obtained, a certain quantity of latex film 110 could be added in order to enhance the mechanical properties thereof, to create a barrier to a possible fibre linting, and to render the surface thereof smoother and more compact.

Preferably, the latex used is based on X-link Vinyl-acetate ethylene copolymer, to be applied in form of foam.

In the structured member 104 according to the present invention, the latex 110 in form of foam is applied to the bottom surface of the layer released by the first forming head 21, in a quantity preferably ranging from 10 to 20 g/m².

The total weight of the structured member 104 may range from 40 to 250 g/m².

The thermal compaction of the various layers released by the forming heads 21, 22, 23, 24 is carried out with a step of exposing said layers to a flow of air heated to a temperature ranging from 120 to 150 °C, thereby forming the integrated member of the invention.

Extensive laboratory tests showed fluids arriving on the top surface of an absorbent member 104 manufactured according to the present invention to be rapidly captured by the special bicomponent fibres located thereat, distributed along a wide planar section of said top surface, and driven towards the central portion thereof.

In said central portion of the absorbent member, the fluids are captured, stored and steadfastly retained due to the high concentration of the absorbent cellulose fibres and of the superabsorbent particles.

Finally, the bottom portion of the absorbent structured member 104 works as a further reservoir for fluids. The latex foam 110 distributed onto the bottom surface of said portion enables a further horizontal diffusion of the fluids stored in said further reservoir.

The present invention has hereto been described according to preferred embodiments thereof, given by way of a non-limiting example. It is understood that other embodiments could be provided, all to be construed as falling within the protective scope thereof, as defined by the appended claims.

## Claims

1. An absorbent article comprising a permeable topsheet (102), an impermeable backsheet (105) and an absorbent body (104) located between said topsheet (102) and said backsheet (105), **characterised in that** said absorbent body (104) is made of a single structured member comprising two or more intermediate layers (111, 112, 113) interconnected by thermal melting, said structured member (104) being thermally fixed to said topsheet (102) and backsheet (105).

2. The article according to claim 1, wherein each intermediate layer of said two or more intermediate layers (111, 112, 113) is manufactured with a mixture of thermoplastic fibres and cellulose fibres.

3. The article according to claim 2, wherein a first intermediate layer (111) of said two or more intermediate layers (111, 112, 113) comprises from the 50 to the 100% of its total weight of thermoplastic fibres.

4. The article according to claim 2, wherein a first intermediate layer (111) of said two or more intermediate layers (111, 112, 113) comprises at least the 90% of its total weight of thermoplastic fibres.

5. The absorbent article according to claim 3 or 4, wherein said thermoplastic fibres of said first intermediate layer (111) have a denier of at least 3 dtex.

6. The absorbent article according to claim 5, wherein said thermoplastic fibres of said first intermediate layer (111) have a length of at least 3 mm.

7. The absorbent article according to claim 3 or 4, wherein said thermoplastic fibres of said first intermediate layer (111) have a denier of 10 dtex.

8. The absorbent article according to claim 7, wherein said thermoplastic fibres of said first intermediate layer (111) have a length of at least 6 mm.

9. The article according to any one of the preceding claims, wherein a second intermediate layer (112) of said two or more intermediate layers (111, 112, 113) comprises at least the 70% of its total weight of thermoplastic fibres.

10. The article according to claim 9, wherein said thermoplastic fibres of said second intermediate layer (112) have a denier of at least 3 dtex.

11. The absorbent article according to claim 10, wherein said thermoplastic fibres of said second intermediate layer (112) have a length of at least 3 mm.

12. The article according to any one of the claims 1 to 8, wherein a second intermediate layer (112) of said two or more intermediate layers (111, 112, 113) comprises at least the 90% of its total weight of thermoplastic fibres.

13. The article according to claim 12, wherein said thermoplastic fibres of said second intermediate layer (112) have a denier of at least 3 dtex.

14. The absorbent article according to claim 13, wherein said thermoplastic fibres of said second intermediate layer (112) have a length of at least 6 mm.

15. The article according to any one of the preceding claims, wherein a third intermediate layer (113) of said two or more intermediate layers (111, 112, 113) comprises at least the 15% of its total weight of thermoplastic fibres.

16. The article according to claim 15, wherein said thermoplastic fibres of said third intermediate layer (113) have a denier of at least 3 dtex.

17. The absorbent article according to claim 16, wherein said thermoplastic fibres of said third intermediate layer (113) have a length of at least 3 mm.

18. The absorbent article according to any one of the claims 2 a 17, wherein said thermoplastic fibres are bicomponent fibres.

19. The article according to any one of the preceding claims, comprising superabsorbent particles (108) located among said two or more intermediate layers (111, 112, 113).

20. The absorbent article according to any one of the preceding claims, wherein said two or more intermediate layers comprise superabsorbent fibres.

21. The article according to any one of the preceding claims, comprising one or more latex film (110), each of said one or more film (110) being located on a respective outer surface of said structured member (104).

22. The article according to claim 21, wherein the latex used is of the X-link Vinyl-acetate ethylene copolymer type, to be applied in form of foam.

23. A process for the manufacturing of an absorbent article (102), **characterised in that** it comprises the steps of:
- providing a permeable topsheet (102);
- providing an impermeable backsheet (105);
- forming a single absorbent body (104) comprising two or more thermally interconnected intermediate layers (111, 112, 113) ;
- placing said absorbent body (104) between said topsheet (102) and said backsheet (105); and
- thermally fixing said absorbent body (104) to said topsheet (102) and to said backsheet (105).

24. The process according to claim 23, wherein said step of forming an absorbent body (104) comprises a step of forming a first intermediate layer (111) comprising from the 50 to the 100% of its total weight of thermoplastic fibres.

25. The process according to claim 24, wherein said thermoplastic fibres of said first intermediate layer (111) have a denier of at least 3 dtex.

26. The process according to claim 25, wherein said thermoplastic fibres of said first intermediate layer (111) have a length of at least 3 mm.

27. The process according to claim 24, wherein said thermoplastic fibres of said first intermediate layer (111) have a denier of 10 dtex.

28. The process according to claim 27, wherein said thermoplastic fibres of said first intermediate layer (111) have a length of at least 6 mm.

29. The process according to any one of the claims 23 to 28, wherein said step of forming an absorbent body (104) comprises a step of forming a second intermediate layer (112) comprising at least the 70% of its total weight of thermoplastic fibres.

30. The process according to claim 29, wherein said thermoplastic fibres of said second intermediate layer (112) have a denier of at least 3 dtex.

31. The process according to claim 30, wherein said thermoplastic fibres of said second intermediate layer (112) have a length of at least 3 mm.

32. The process according to any one of the claims 23 to 28, wherein said step of forming an absorbent body (104) comprises a step of forming a second intermediate layer (112) comprising at least the 90% of its total weight of thermoplastic fibres.

33. The process according to claim 32, wherein said thermoplastic fibres of said second intermediate layer (112) have a denier of at least 3 dtex.

34. The process according to claim 33, wherein said thermoplastic fibres of said second intermediate layer (112) have a length of at least 6 mm.

35. The process according to any one of the claims 23 a 34, wherein said step of forming an absorbent body (104) comprises a step of forming a third intermediate layer (113) comprising at least the 15% of its total weight of thermoplastic fibres.

36. The process according to claim 35, wherein said thermoplastic fibres of said third intermediate layer (113) have a denier of at least 3 dtex.

37. The process according to claim 36, wherein said thermoplastic fibres of said third intermediate layer (113) have a length of at least 3 mm.

38. The process according to any one of the claims 24 to 37, wherein said thermoplastic fibres are bicomponent fibres.

39. The process according to any one of the claims 23 to 38, further comprising a step of introducing superabsorbent particles (108) between two or more of said intermediate layers (111, 112, 113).

40. The process according to any one of the claims 23 to 39, further comprising a step of applying one or more latex film (110), at respective outer surfaces of said structured member (104).

41. The process according to claim 40, wherein the latex used is of the X-link Vinyl-acetate ethylene copolymer type, to be applied in form of foam.

42. The process according to claim 40 or 41, wherein said one or more latex film (110) has a density ranging from 10 to 20 g/m^{2.}
